# EUROPEAN PATENT APPLICATION

(11) **EP 4 744 695 A1**
(43) Date of publication of application: **20.05.2026**
(21) Application number: 24306905.1
(22) Date of filing: 14.11.2024
(51) Int. Cl.: A61M 5/00, A61M 5/28, A61M 5/32

(54) **REUSABLE ADD-ON CASING FOR AN INJECTION DEVICE**

(71) Applicant: Becton Dickinson France, 38800 Le Pont-de-Claix (FR)
(72) Inventor: DELOBELLE, Vincent, 38420 DOMENE (FR); FIARD, Michael, 38320 Eybens (FR); BERNÈDE, Gilles, 74930 Arbusigny (FR); BOUYAHIAOUI, Salim, 74000 ANNECY (FR); GAUTHIER, Aurélien, 74000 Annecy (FR)
(74) Representative: Germain Maureau

(57) **Abstract**

This casing (1) includes: a body (2) delimiting an inner cavity for receiving the injection device (5), the body (2) including a proximal end (204) and a distal end (202) configured for axially abutting against an injection site; a proximal stop (22) configured for blocking distal movement of the injection device (5) at a predetermined axial position within the body (2); a locker (3) for axially blocking the injection device (5) within the body (2), the locker (3) being movable with respect to the body (2) between an unlocked position allowing insertion or removal of the injection device (5), and a locked position in which the locker (3) prevents removal of the injection device (5) from the body (2); and a cap (4) removably attached to the body (2), the cap (4) including a gripping member (43) configured for gripping a needle shield (54) of the injection device (5), such that removal of the cap (4) from the body (2) entails removal of the needle shield (54) from the injection device (5), and a release element (44) configured for releasing the needle shield (54).

## Description

The present invention relates to a reusable add-on casing for an injection device.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction away from the user's hand, and the "proximal direction" is to be understood as meaning the direction toward the user's hand.

In this application, inner or inward means oriented towards a central longitudinal axis A, and outer or outward means oriented opposite to the central longitudinal axis A.

It is known to use injection devices including a syringe having an injection needle and a barrel designed for containing a medical product, a body for receiving the syringe, a guard for covering the injection needle after injection, a plunger rod to dispense the medical product from the syringe and a spring for activation of the guard. The guard and the body are held together by two trigger fingers prior to use of the device. At the end of injection, the plunger rod abuts against the trigger fingers and deflect the trigger fingers such that the guard is released from the body. Under the action of the spring, the guard distally moves to its safety position.

When using this injection device, the user needs to prick a patient at an appropriate skin depth. Inserting the injection needle at an inappropriate needle exposure length may result in injecting the medical product in the wrong tissues, for instance in muscular tissues instead of subcutaneous tissues.

There is therefore a need for a device that permits to control the needle exposure length. There is also a need for a device that is ergonomic so that even untrained users would be able to perform injection properly. There is also a need for a device that is reusable to limit the environmental impact.

In this context, an aspect of the invention is a casing configured for receiving an injection device, the casing including:
a body delimiting an inner cavity for receiving the injection device, the body including a proximal end and a distal end configured for axially abutting against an injection site,
a proximal stop configured for blocking distal movement of the injection device at a predetermined axial position within the body,
a locker for axially blocking the injection device within the body, the locker being movable with respect to the body between an unlocked position allowing insertion or removal of the injection device, and a locked position in which the locker prevents removal of the injection device from the body, and
a cap removably attached to the body, the cap including a gripping member configured for gripping a needle shield of the injection device, such that removal of the cap from the body entails removal of the needle shield from the injection device, and a release element configured for releasing the needle shield.

The casing of the invention thus permits to control the needle exposure length. Besides, thanks to the locker that can release the injection device, and to the cap that can release the removed needle shield, the casing can be re-used. The casing is an add-on device, capable of being removably added to an injection device in order to add or improve functions of the injection device. The used injection device can be withdrawn from the casing of the invention after moving the locker to the unlocked position. The needle shield can further be released from the cap by the release element of the cap. The cap can then be mounted back onto the casing. The casing of the invention is re-usable.

The casing of the invention may further include some or all of the features listed below.

The proximal stop may be delimited by the body.

In an embodiment, the proximal stop is arranged at the proximal end of the body.

In an embodiment, the body includes a finger flange for providing support to a user's fingers. The finger flange may be arranged at a proximal end of the body.

In an embodiment, the finger flanger includes a guiding member for guiding movement the locker.

Possibly, the finger flange is circular or rectangular.

Possibly, the body has a cylindrical or a parallelpipedic shape. The body may thus have a circular or a square or a rectangular, or even a polygonal cross-section shape.

Possibly, the locker is permanently coupled to the body. More specifically, the locker is permanently coupled to the guiding member. That is, in both the unclocked and the locked positions. By permanently it is meant that the locker and the body are not intended to be disassembled during use of the casing.

Alternatively, the locker is attached to the body in the locked position and is detached from the body in the unclocked position. The locker and the body can thus be separated. The locker and the body are here designed to be disassembled during use of the casing. The locker is an add-on part configured to be coupled to or removed from the body.

In an embodiment, the locker is radially, rotationally or axially movable with respect to the body.

Possibly, the locker is configured for being moved to the locked position or to the unlocked position by the user.

In an embodiment, the cap is made of a single piece.

In an embodiment, the cap is made of two assembled parts: (i) an attachment portion configured to be removably attached to the body, and (ii) the release element.

The attachment portion may include the gripping member.

In an embodiment, the gripping member is movable between a gripping position in which the gripping member can grip the needle shield of the injection device and a release position in which the gripping member can release the needle shield.

In an embodiment, the release element is movable between a first position in which the release member allows the gripping member to grip the needle shield and an ejection position in which the release member moves the gripping member to the release position.

The release element is movable with respect to the gripping member and/or the attachment portion.

In an embodiment, the release element is rotationally movable with respect to the gripping member. The release element may rotate around the longitudinal axis A. The release element may simultanesously be axially fixed with respect to the gripping member.

In an embodiment, the release element is axially movable with respect to the gripping member. The release element may simultaneously be rotationally fixed with respect to the gripping member.

In an embodiment, the release element is both rotationally and axially movable with respect to the gripping member. The release element may be connected to the gripping member by means of a helical slot.

In an embodiment, the release element is radially movable with respect to the gripping member. The release element may simultaneously be axially and rotationally fixed with respect to the gripping member.

In an embodiment, the casing includes a motion transmitter arranged between the locker and the gripping member such that movement of the locker from the unlocked position to the locked position entails movement of the gripping member from the release position to the gripping position. Similarly, the motion transmitter transforms movement of the locker from the locked position to the unlocked position into movement of the gripping member from the gripping position to the release position.

Possibly, the cap, more specifically the attachment portion and/or the release element, has a cylindrical, a parallelepipedic or a polygonal shape.

Another aspect of the invention is a drug delivery device including the aforementioned casing and an injection device arranged within the casing.

The injection device may include a medical container provided with an injection needle, and a safety device configured for receiving the medical container and for protecting a user against needle stick injuries.

Another aspect of the invention is a cap configured to be removably attached to a body of a casing configured for receiving an injection device, the cap including a gripping member configured for gripping a needle shield of the injection device, such that removal of the cap from the body entails removal of the needle shield from the injection device, and a release element configured for causing the gripping member to release the needle shield.

Thus, the cap enables to discard the used needle shield in a waste container such that the cap can be re-used with another injection device.

The cap may further include some or all of the aforementioned features.

The invention and the advantages arising therefrom will clearly emerge from the detailed description that is given below with reference to the appended drawings as follows :
- Figure 1 is a cross-section view of a safety device of a drug delivery device according to an embodiment of the invention,
- Figure 2 is a perspective view of a medical container of a drug delivery device according to an embodiment of the invention,
- Figures 3A and 3B are perspective views of a drug delivery device including a housing according to an embodiment of the invention, respectively on a locked position and in a release position,
- Figure 3C is a cross-section view of the drug delivery device of Figure 3A,
- Figures 4A and 4B are top views of drug delivery device of, respectively, Figures 3A and 3B,
- Figure 4C is a cross-section view illustrating the positions of the locker of the drug delivery device of Figures 4A and 4B, the upper part of Figure 4C being the release position, the lower part of Figure 4C being the locked position,
- Figure 5A, 5B and 5C are perspective views of a housing of a drug delivery device according to another embodiment of the invention, the locker being in the release position in Figure 5B and in the locked position in Figures 5B and 5C,
- Figure 5D is a cross-section view of the drug deliver device of Figure 5C,
- Figure 6A and 6B are perspective views of a drug delivery device including a housing according to an embodiment of the invention, respectively on a locked position and in a release position,
- Figure 7A illustrates a body of the housing of a drug delivery device according to another embodiment of the invention,
- Figure 7B is a perspecive view of a locker of the housing of a drug delivery device according to this embodiment of the invention,
- Figure 7C illustrates the locker of Figure 7B in the locked position,
- Figures 8A and 8B are perspective views of a drug delivery device including a housing according to an embodiment of the invention, respectively on a locked position and in a release position,
- Figure 9A is a perspective view of a cap of the housing according to an embodiment of the invention,
- Figures 9B and 9C are partial cross-section views illustrating the gripping member of the cap of Figure 9A in, respectively, the gripping position and the release position,
- Figure 9D is a cross-section view of the cap of Figure 9A,
- Figures 10A-10F are views of a cap of a housing according to another embodiment of the invention,
- Figures 11A-11C are views of a cap of a housing according to another embodiment of the invention,
- Figures 12A and 12B are views of a cap of a housing according to another embodiment of the invention,
- Figures 13A to 13G are views of a cap of a housing according to another embodiment of the invention,
- Figure 14A is a perspective view of a drug delivery device according to another embodiment of the invention, the locker being in the release position,
- Figures 14B and 14C are cross-section views of the drug delivery device of Figure 14A,
- Figure 14D is a perspective view of a locker of the drug delivery device of Figure 14A,
- Figure 14E is a top view of the drug delivery device of Figure 14A, the locker being removed for the skae of clarity,
- Figure 14F is a perspective view of a body of the drug delivery device of Figure 14A,
- Figure 14G is a perspective view of the drug delivery device of Figure 14A, the locker, the attachment portion of the cap and the body being removed for the sake of clarity,
- Figure 14H is a partial cross-section view of an attachment portion of the cap of the drug delivery device of Figure 14A,
- Figure 14I is a perspective view of a release element of a cap of the drug delivery device of Figure 14A,
- Figures 15A to 15F illustrate operation of a housing according to an embodiment of the invention.
- 6426

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. In contrast, when an element is referred to as being "directly connected" or "directly coupled" to another element, there are no intervening elements present. Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein. Further, terms such as distal, proximal, up, down, bottom, and top are relative, and are to aid illustration, but are not limiting. Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure. As used herein, the singular forms "a," "an," and "the" are intended to include the plural forms as well, unless the context clearly indicates otherwise. As used herein, the term "and/or" includes any and all combinations of one or more of the associated listed items. It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure. For simplification, the parts or elements of one embodiment which are found identically or similarly in the other embodiment will be identified using the same numerical references and will not be described again.

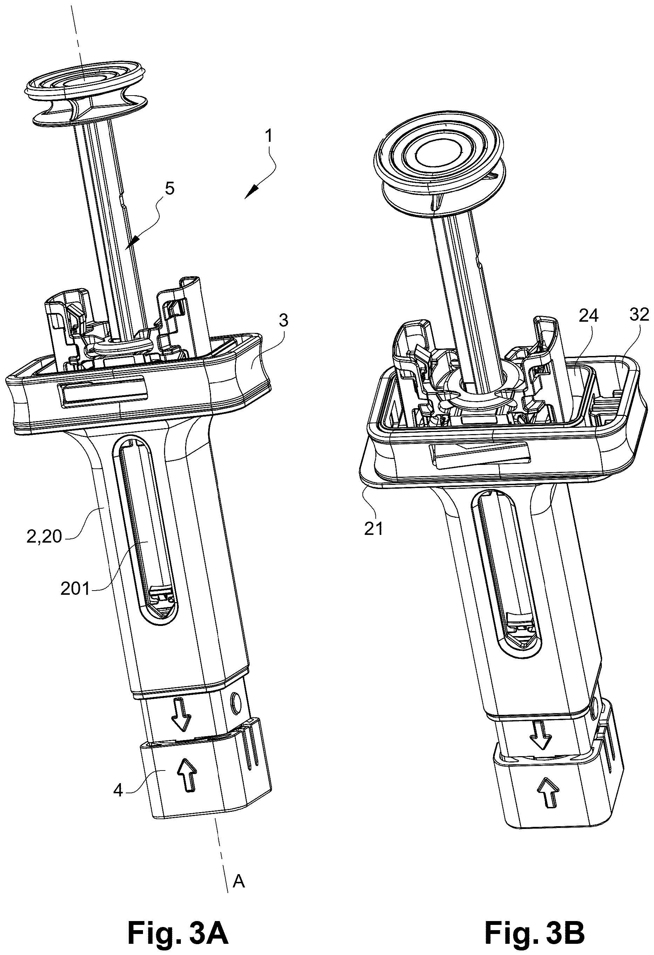

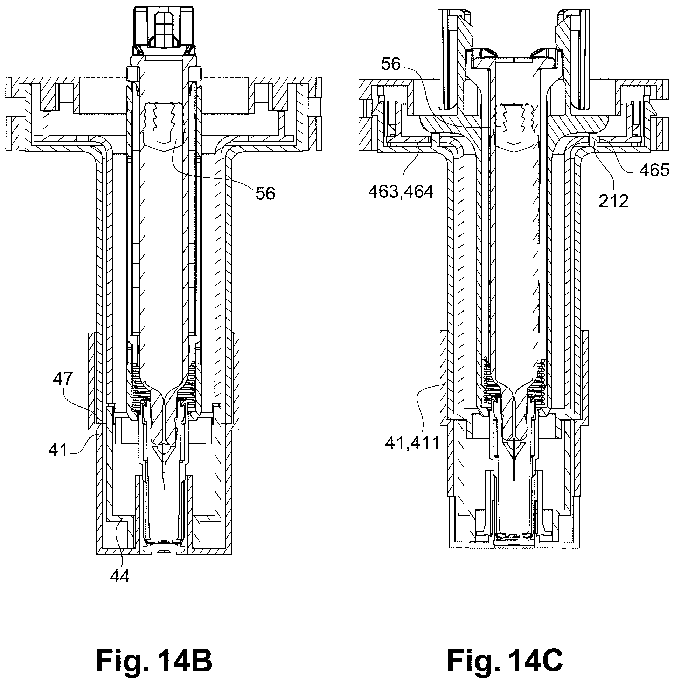

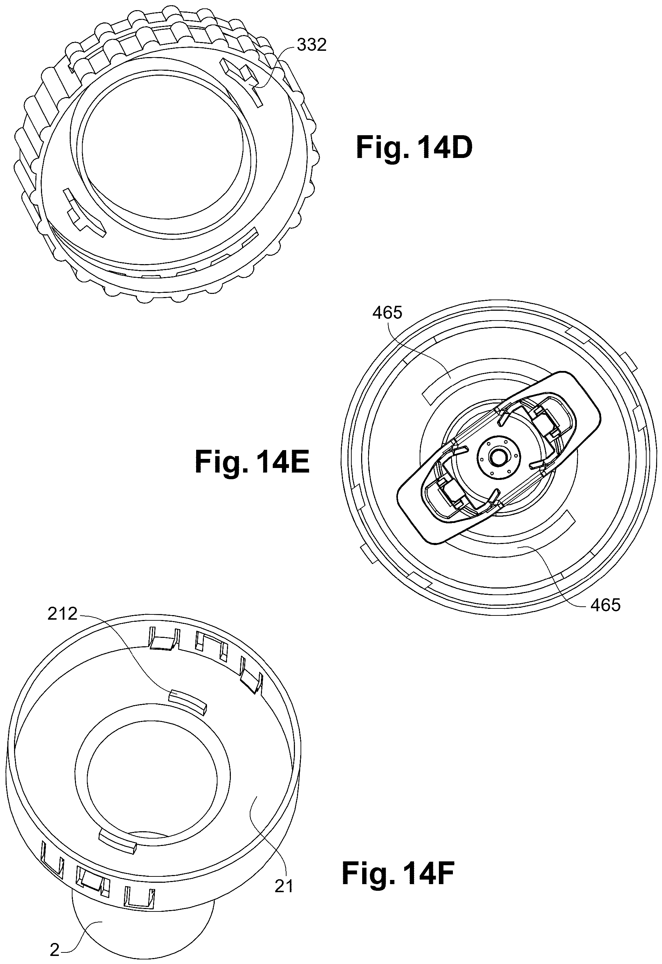

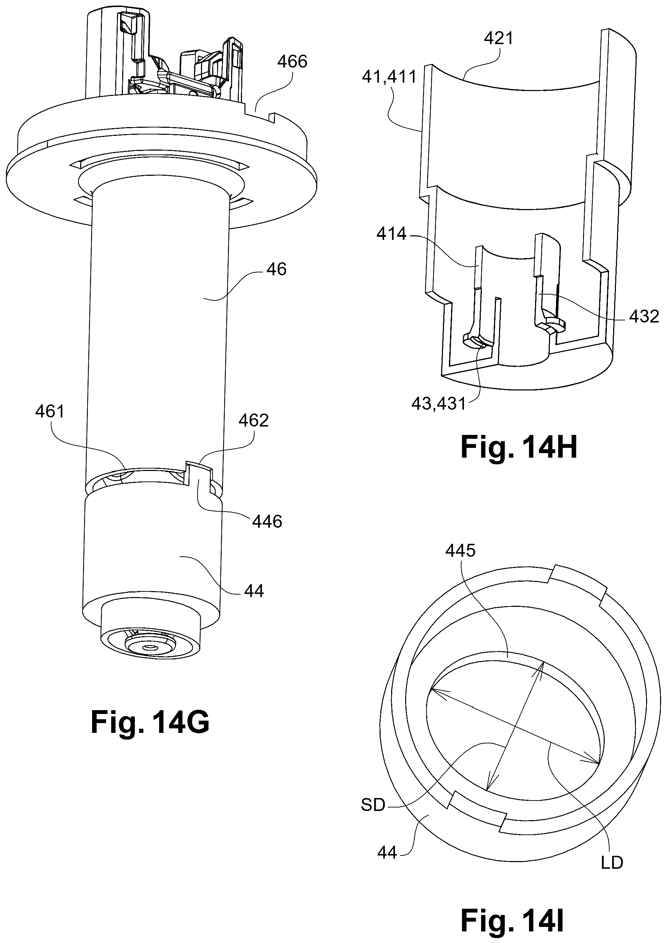

With reference to Figure 3A is shown a casing 1 according to an embodiment of the invention. The casing 1 is configured for housing an injection device 5. The injection device 5 may include a medical container 51, such as a prefillable or prefilled syringe. The medical container 51 includes a barrel 52 defining a reservoir for containing a medical product. The barrel 52 may include a longitudinal distal tip 521 to which is attached an injection needle 53 fluidly connected to the reservoir. A needle shield 54 is removably attached to the distal tip 521 to protect and seal the injection needle 53 before use. A plunger rod 55 is configured for extending through an opened proximal end 522 of the barrel 52 to push a stopper 56, Figures 14B-14C, arranged within the barrel 52, thereby expelling the medical product outside the medical container 51 via the distal tip 521 and the injection needle 53.

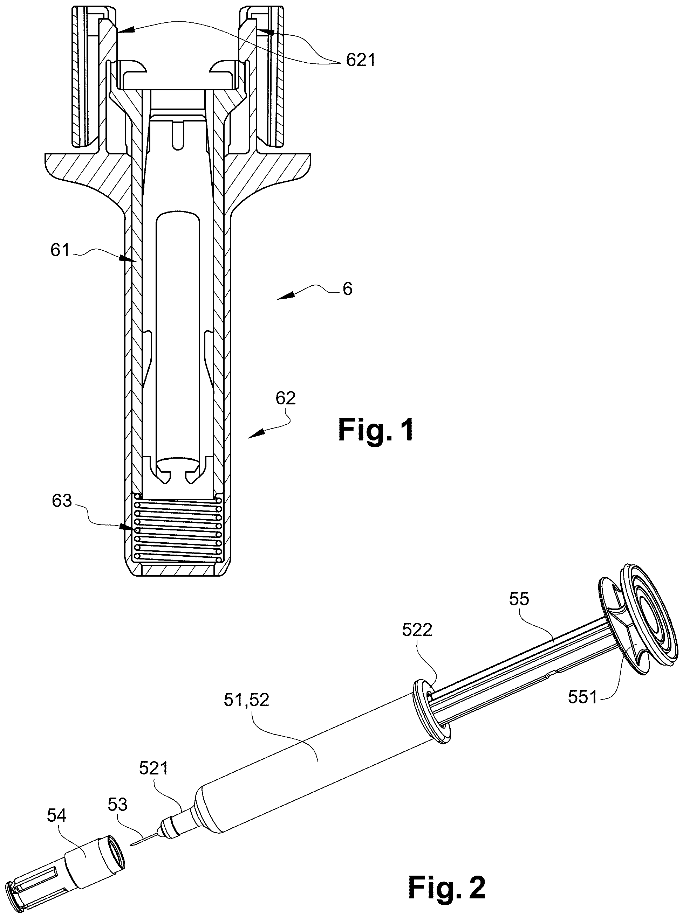

The medical container 51, illustrated in Figure 2, may be contained within a safety device 6 of the type illustrating in Figure 1, including a body 61 for receiving the medical container 51, a guard 62 for covering the injection needle 53 after injection, and a spring 63 for activation of the guard. The guard and the body are held together by two trigger fingers 621 prior to use of the device. At the end of injection, the plunger rod 55 abuts against the trigger fingers 621 and deflect the trigger fingers 621 such that the guard 62 is released from the body 61 and can deploy in the distal direction, under the action of the spring 63, to cover the injection needle 53.

The casing 1 of the invention may include a outer body 2 for receiving the injection device 5, a locker 3 for locking the injection device 5 within the outer body 2, and a removable cap 4 for detaching a needle shield 54 of the injection device 5.

The outer body 2 extends along a longitudinal axis A and around the injection device 5. The outer body 2 may in the form of a tubular sleeve, having a cylindrical, elliptic, parallelepipedic or polygonal shape allowing for ergonomic seizing of the casing 1 by the user. The outer body 2 has a lateral wall 20 defining an inner cavity for containing the injection device 5. The lateral wall 20 may include a see-through window 201 allowing for visual inspection of the medical container 51.

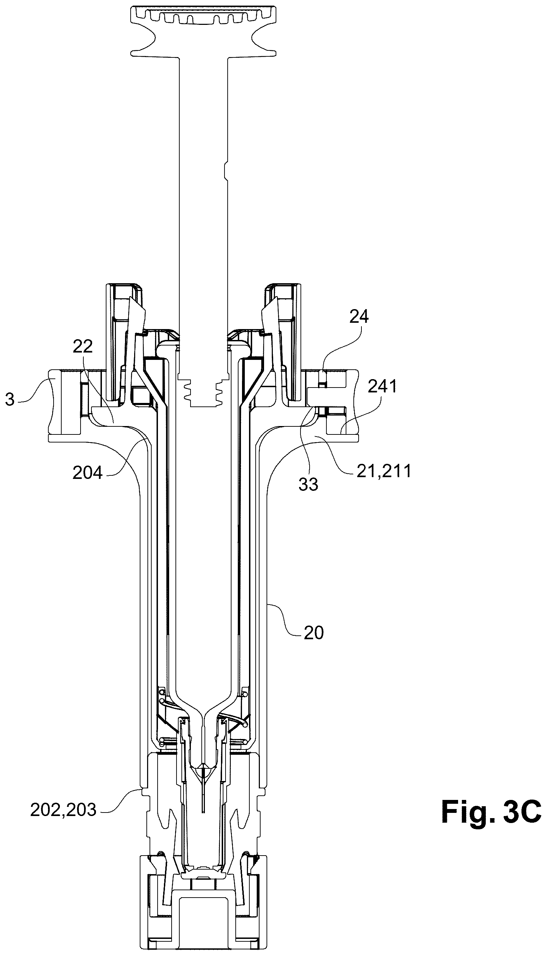

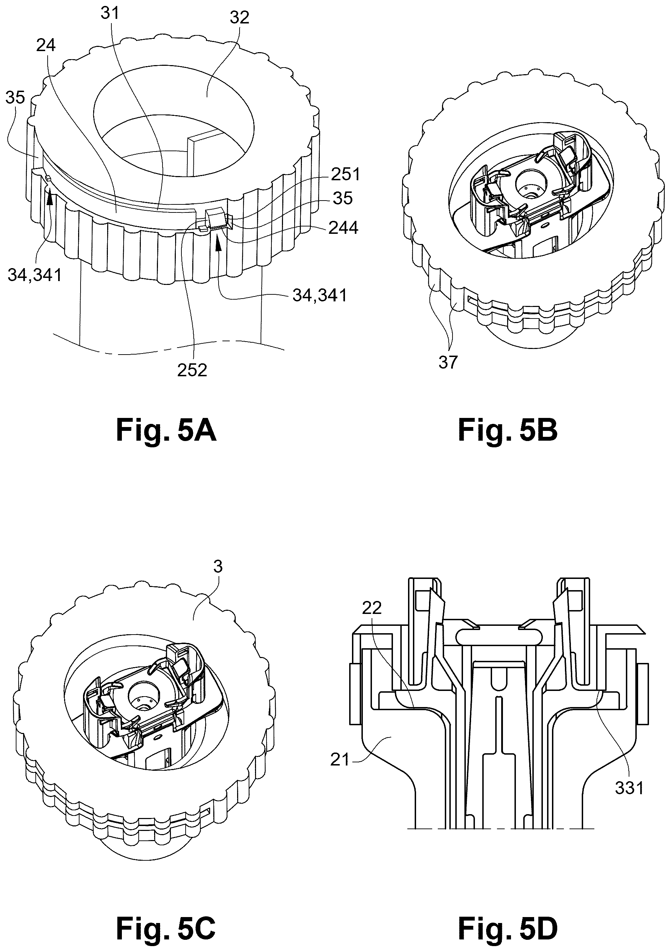

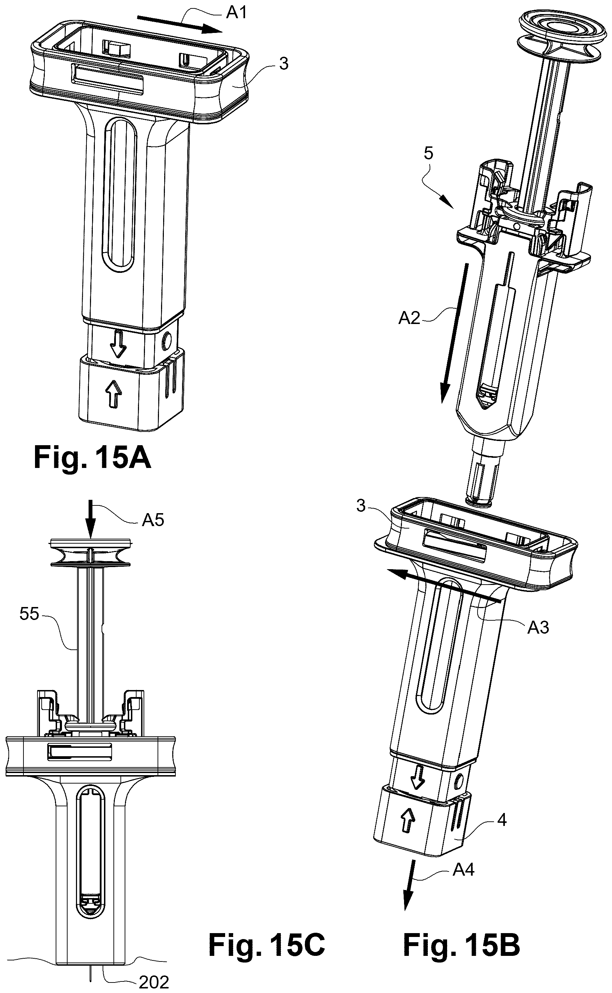

With reference to Figure 3C, the outer body 2 has an opened distal end 202 configured for axially abutting against the injection site (see Figures 3C and 15C), thereby contributing to control the needle exposure length. The distal end delimits a distal opening 203 configured for allowing extension of the guard 62 of the injection device 5 after injection. Opposite the distal end 202, the outer body 2 has an opened proximal end 204 for insertion of the injection device 5. The outer body 2 has a finger flange 21, which may radially outwardly protrude at the opened proximal end 204 of the outer body 2, for providing ergonomic support to the user's fingers. In one embodiment, the finger flange 21 may have a rectangular shape, defined by two diametricallly opposite radial extensions 211. In another embodiment, Figures 5A-5D, the finger flange 21 may have a circular or oval shape. In other embodiments (not shown), the finger flange 21 may have a square or any other polygonal shape. The finger flange 21 may thus extend all around the proximal end 204 of the outer body 2, and not solely at the two radial extensions 211. The finger flange 21 is designed to avoid hindrance of the plunger rod 55 distal movement and to avoid hindrance of outward deflection of the trigger fingers 621, i.e. to avoid hindrance of activation of the trigger fingers 621 by the plunger rod 55 at the end of injection.

The outer body 2 includes a proximal stop 22, which may be arranged at the proximal end 204 of the outer body 2 and/or be defined by a proximal side of the finger flange 21, for axially abutting against the injection device 5. For instance, the proximal stop 22 of the outer body 2 axially abuts against a finger flange 21 of the injection device 5. The proximal stop 22 may be a peripheral surface that surrounds a proximal opening delimited at the proximal end 204 of the outer body 2. The proximal stop 22 allows for preventing distal movement of the injection device 5 with respect to the casing 1 of the invention. Taking in account the axial length of the injection needle 53, the relative axial position of the proximal stop 22 and of the distal end 202 of the outer body 2 defines a predetermined needle exposure length. In other words, knowing the axial length of the injection needle 53, the axial length separating the proximal stop 22 from the distal end 202 of the outer body 2 defines the predetermined needle exposure length. Since the needle exposure length is physically determined by the outer body 2, the user can reliably prick at the right injection depth, and therefore at the right tissues, without requiring high skills. The outer body 2 may also include orthoradial abutments for rotationally securing the injection device 5 with respect to the casing 1, so that the injection device 5 cannot rotate within the casing 1. The orthoradial abutments thus orthoradially abut against the injection device 5 when the injection is positioned within the casing 1.

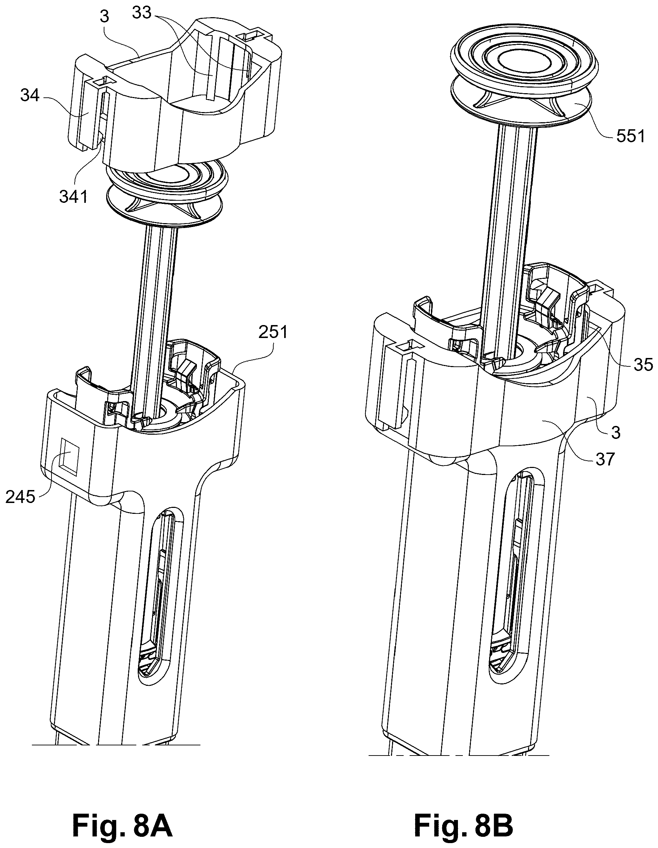

The outer body 2 further includes a guiding member 24, which may extend proximal to the finger flange 21, for guiding movement of the locker 3 with respect to the outer body 2. The guiding member 24 may be in the form of a skirt, proximally protruding from the finger flange 21 of the outer body 2. In embodiments, the skirt 24 and the finger flange 21 may delimit a proximal shoulder 241 on which the locker 3 is designed to slide. The skirt 24 has a lateral wall 242 whose outer surface may be configured to guide movement of the locker 3. In other embodiments, Figures 5A to 7C, it is not the outer surface but guiding apertures 243 provided through the lateral wall 242 of the skirt that are configured to receive and guide the locker 3. The skirt 24 and the locker 3 may have complementary shapes, such as that the locker 3 may axially (Figures 8A-8B), radially (Figures 3A-4C, 6A-6B, or 7A-7C) or rotationally (Figures 5A-5D) slide with respect to the outer body 2.

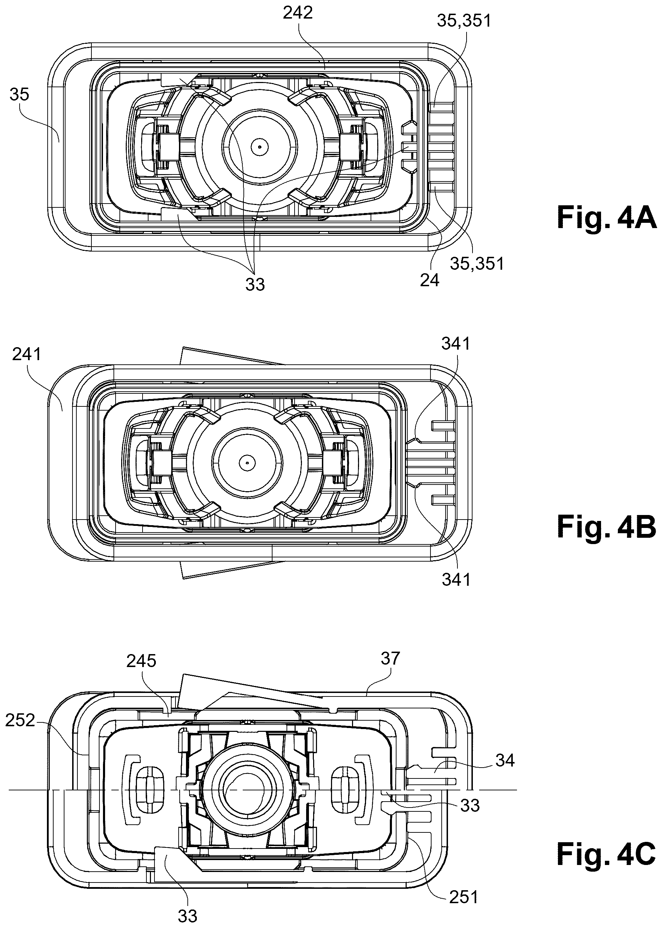

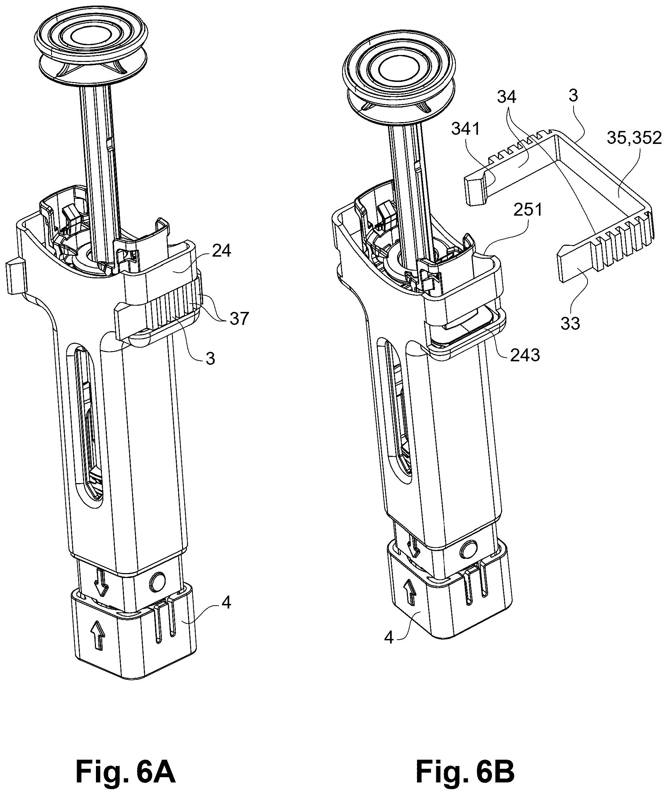

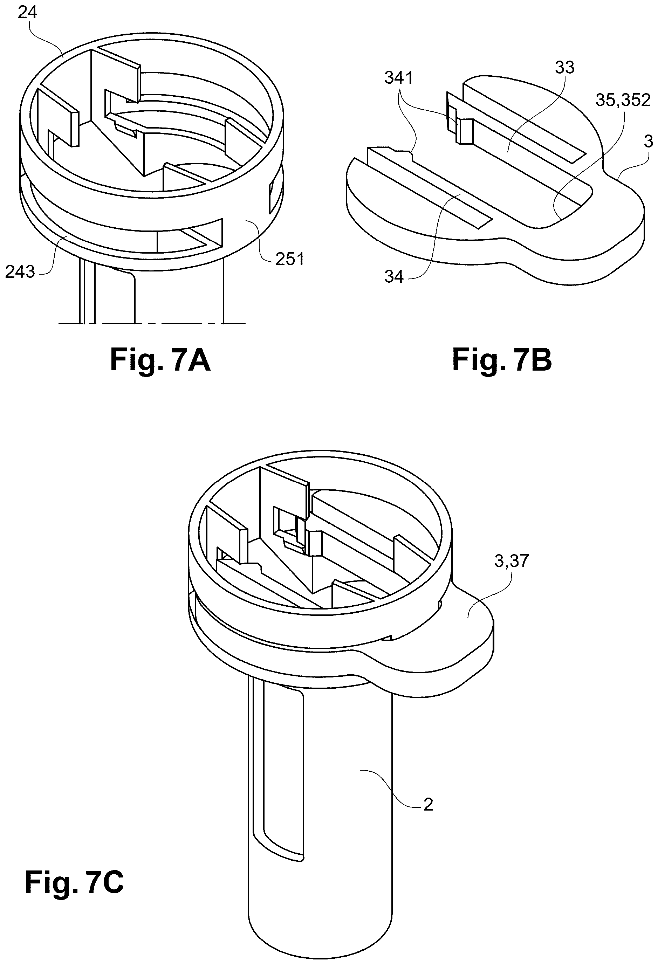

In embodiments, the outer body 2 may have one or more first stops 251 for stopping movement of the locker 3 when the locker 3 reaches the locked position, and/or one or more second stops 252 for stopping movement of the locker 3 when the locker 3 reaches the unlocked position. In embodiments, Figure 4C, the one or more first stops 251 may be defined by a lateral side of the outer surface of the skirt 24 and/or, Figure 8A, by an edge of one or more apertures 245 provided through the skirt 24, while the one or more second stops 252 may be defined, Figure 4C, by a diametrically opposite side of the outer surface of the skirt 24. In other embodiments, Figures 5A-5D, the first stop 251 and the second stop 252 may be opposite orthoradial surfaces of an outward radial protrusion of a proximally extending resilient leg 244 provided through the lateral wall 242 of the skirt 24 for engaging a circumferential opening 31 of the locker 3. In embodiments, Figures 6A-6B and 7A-7C, the first stop 251 may be a lateral side of the outer surface of the skirt 24. In another embodiment, Figures 8A-8B, the first stop 251 may be a proximal end of the skirt 24.

The outer body 2, the finger flange 21 and/or the skirt 24 may be made of a single piece.

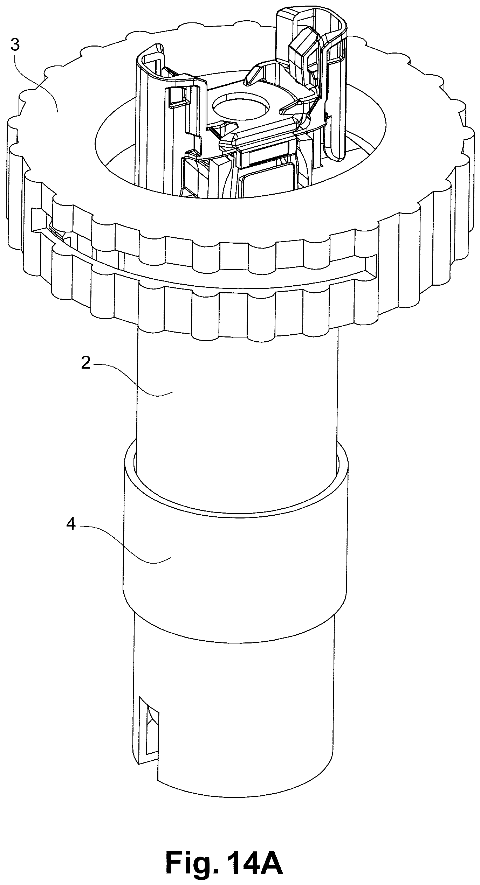

The locker 3 may be arranged at the proximal end 204 of the outer body 2 for axially securing the injection device 5 within the outer body 2. The locker 3 may be in the form of a rectangular ring (Figures 3A-4C) or a circular ring (Figures 5A-5D and 14A) or any closed ring of other appropriate shape complementary to the skirt 24 of the outer body 2 and allowing relative sliding (axial, radial, or rotational) movement between the locker 3 and the outer body 2. The locker 3 may delimit an opening 32. In some embodiments, Figures 5A-5D and 14A, the opening 32 has a non-circular shape, such as an oblong shape. Therefore, rotation of the locker 3 either permits or forbids passage of the injection device 5 through this opening 32.

The locker 3 is indeed movable with respect to the outer body 2 between an unlocked position, Figures 3B, 4B, 5B, 6B, 7A-7B, 8A in which the locker 3 allows for axial insertion or removal of the injection device 5, and a locked position, Figure 3A, 4A, 5C, 6A, 7C, 8B in which the locker 3 axially blocks the injection device 5 within the outer body 2. That is, the injection device 5 is distally blocked by the proximal stop 22 of the outer body 2 and one or more distal abutment members 33, Figures 3C and 4C, of the locker 3. The distal abutment member 33 may be configured for axially abutting against a proximal side of the finger flange 21 of the injection device 5. In embodiments, Figures 4A and 4C, the distal abutment member 33 of the locker 3 may be an inwardly radially extending rod, and/or one or more tangential resilient legs which are configured for returning to their rest position in which they extend on the axial path of the injection device 5. In this embodiment, the locker 3 has three distal abutments members 33 forming a triangle inluding the central longitudinal axis A. The resilient legs may be symmetrical with respect to a longitudinal plane including the longitudinal axis A. The resilient legs engage lateral apertures 245 of the skirt of the outer body 2. In other embodiments, Figure 5D the distal abutment member 33 may be a distal end of a distally extending skirt 331, which may have an oblong shape so that two diametrically opposite portions of the skirt can abut against the injection device 5. In other embodiments, Figures 6A-6B and 7A-7C, the distal abutment member 33 may be defined by parallel legs of a U-shaped or V-shaped locker 3. In other embodiments, Figure 8A, the distal abutment member 33 may be a distal end of one or more, for instance four, axially extending ribs.

It is of note that the locker 3 may be either radially movable with respect to the outer body 2 i.e. orthogonal to the longitudinal axis A, see Figures 3A-3B, 6A-6B, 7A-7C, or rotationally movable with respect to the outer body 2 around the longitudinal axis A, see Figures 5B-5C, or axially movable with respect to the outer body 2 along the longitudinal axis A, see Figures 8A-8C. Anyway, movement of the locker 3 from the unlocked position to the locked position, and vice versa, may be caused by the user.

The locker 3 may include one or more snap-fit members 34 configured for removably attaching the locker 3 to the outer body 2, or for retaining the locker 3 in the locked and/or unlocked position. In the embodiment illustrated in Figure 4B, the snap-fit members34 are two inward radial resilient legs including a protrusion 341. One side of the protrusion radially abuts against the skirt 24 of the outer body 2 in the unlocked position, while an opposite side of the protrusion 341 radially abuts against the skirt 24 of the outer body 2 in the locked position. In the embodiment illustrated in Figure 5A, the one or more snap-fit members 34 include a first and a second protrusions 341 arranged in the circumferential opening 31 of the locker 3, respectively at two opposite ends of this circumferential opening 31, to cooperate with the resilient leg 244 of the skirt to retain the locker 3 in the unlocked position and in the locked position. In the embodiment illustrated in Figure 6B, the snap-fit members 34 are two resilient legs provided with a protrusion 341 for engaging the outer surface of the skirt 24 of the outer body 2. In Figures 7A-7C, the snap-fit members 34 are resilient legs provided with a protrusion 341 for engaging lateral apertures 243 of the skirt of the outer body 2. In the embodiment illustrated in Figure 8A, the snap-fit members 34 are two axial resilient legs provided with a protrusion 341 for engaging lateral apertures 245 of the skirt of the outer body 2. Thus, the locker 3 can have two stable positions.

The locker 3 may have one or more abutments 35 configured for abutting against the skirt 24 of the outer body 2 in the unlocked and/or locked positions in order to stop movement of the locker 3. In the embodiment of Figure 4A, the locker 3 has a radial abutment 35 formed at a side of its lateral wall 36 for radially abutting against a side of the skirt, and an opposite radial abutment formed at a free end of two radial inward tabs 351 for radially abutting against an opposite side of the skirt. In the embodiment of Figure 5A, the locker 3 has two opposite orthoradial abutments 35 at the two ends of the circumferential opening 31. In the embodiment of Figures 6B and 7B, the locker 3 has a radial abutment 35 arranged at an inner side of an intermediate arm 352 connecting the snap-fit members 34 of the U- or V-shaped locker 3 for radially abutting against the skirt of the outer body 2. In the embodiment illustrated in Figure 8B, the locker 3 has a distal abutment 35 for axially abutting against the proximal end of the skirt 24.

The locker 3 may have one or more grasping elements 37 arranged on an outer surface of the locker 3 for allowing the user to grasp and thus displace the locker 3 between the unlocked and locked positions. In the embodiment of Figures 4A-4C, the grasping element 37 includes a side surface of the locker 3. This side surface may have a concave shape. In the embodiment illustrated in Figure 5B, the grasping elements 37 include a plurality of axial ribs outwardly protruding from the outer lateral surface of the locker 3. The axial ribs may may be regularly distributed all around the locker 3 to ease rotation of the locker 3. In the embodiment of Figure 6A, the grasping elements 37 also include a plurality of parallel axial ribs arranged at an outer surface of the snap-fit members 34 for easing radial movement of the locker 3 with respect to the skirt 24 of the outer body 2. In the embodiment illustrated in Figure 7C, the grasping element 37 is a radially extending wing that sticks out the outer body 2 so that the user can grasp it and pull the locker 3 in the radial direction. In the embodiment of Figure 8B, the grasping elements 37 may be two diametrically opposite sidewalls of the locker 3 which have a convex shape for passing over a flange 551 of the plunger rod 55 and which ease grasping of the locker 3 so that the user can pull the locker 3 in the axial direction.

The one or more distal abutment members 33, and/or snap-fit members 34, and/or abutments 35, and/or grasping elements 37 may preferably be made of a single piece. That is, the locker 3 is preferably made of a single piece.

The locker 3 may be separate from the outer body 2, Figures 6A-6B, 7A-7C, 8A-8B or may be connected to the outer body 2, Figures 3A-3, 4A-4C, 5A-5D, 14A. In either case, the locker 3 and the outer body 2 are distinct parts, i.e. are not made of a single piece.

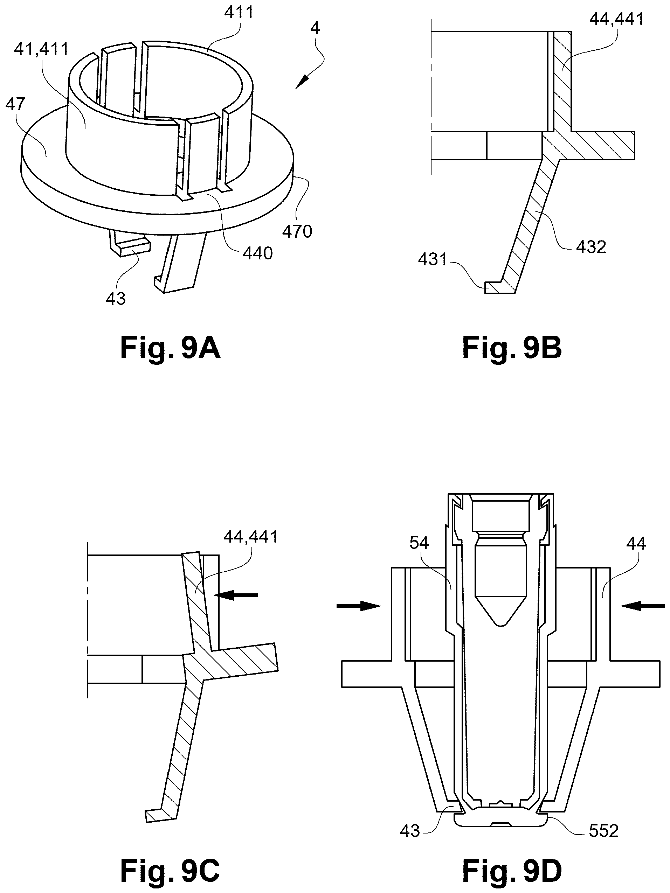

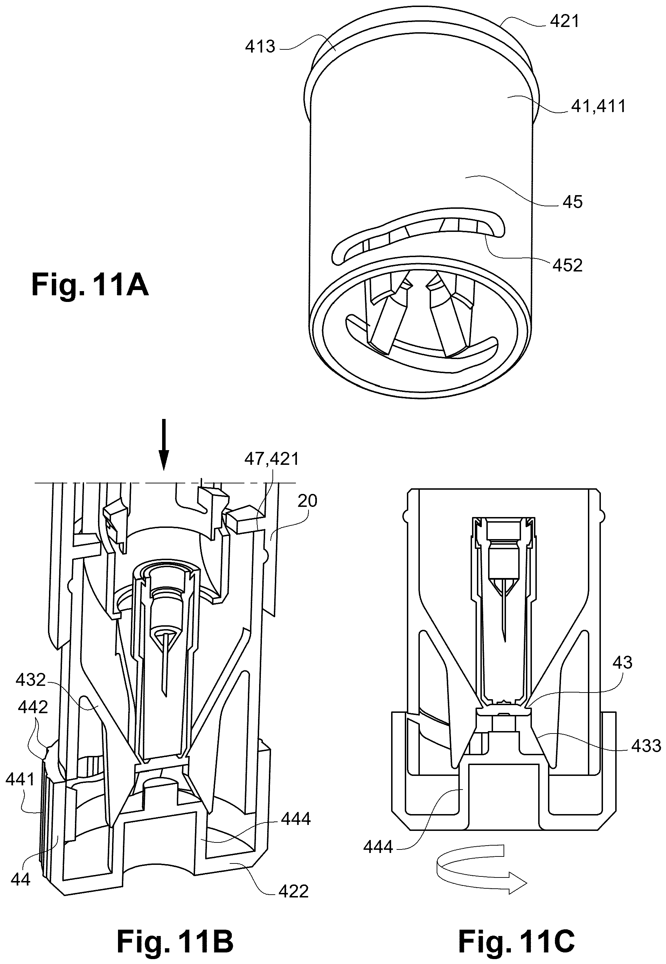

The casing 1 includes a cap 4 which may be removably attached to the distal end 202 of the outer body 2 for protecting the needle shield 54 and the injection device 5 before use. The cap 4 is configured for being removed from the outer body 2 by pulling the cap 4 in the distal direction. The cap 4 includes one or more attachment portions 41, which may be arranged at a proximal end 421 thereof, for removably attaching the cap 4 to the distal end 202 of the outer body 2. The attachment portions 41 may allow for instance a snap-fit or a friction-fit connection between the cap 4 and the outer body 2. In the embodiment illustrated in Figure 9A, the attachment portions 41 may be in the form of two hemicylindrical sidewalls 411 configured for frictionnally engaging an inner surface of the lateral wall 20 of the outer body 2. In the embodiment illustrated in Figure 9A, 10A, 13A or 11A-12B, the attachment portion 41 is a square or cylindrically shaped sidewall 411 configured for frictionnally engaging an inner surface of the lateral wall 20 of the outer body 2. Any other shape capable of frictionnally engage the inner surface of the lateral wall 20 of the outer body 2 may be appropriate. The attachment portion 41 may further include one or more axial ribs 412 outwardly protruding from the sidewall 411 for increasing friction between the outer body 2 and the cap 4. In another embodiment illustrated in Figures11A-12B, the attachment portion 41 includes a cylindrical sidewall 411, which may be arranged at the proximal end 421 of the cap 4, for frictionnally engaging an inner surface of the lateral wall 20 of the outer body 2. The attachment portions 41 may further include a circumferential rib (or groove) 413 for complementarily engaging a circumferential groove (or rib) of the outer body 2. In the embodiment illustrated in Figure 14H, the attachment portion 41 may include a cylindrical sidewall 411 arranged at a proximal end 421 of the cap 4 for frictionally engaging an outer surface (instead of the inner surface) of the lateral wall 20 of the outer body 2.

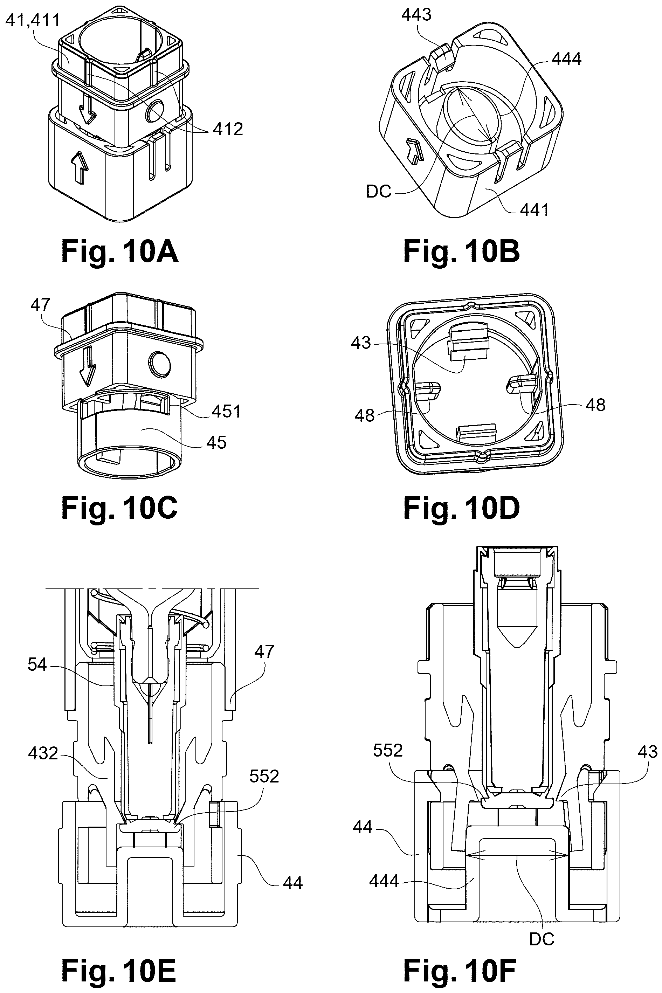

The cap 4 further includes one or more gripping members 43 configured for engaging the needle shield 54 such that removal of the cap 4 from the outer body 2 entails removal of the needle shield 54 from the medical container 51 of the injection device 5. The gripping member 43 may engage a lateral wall, a proximal end, or a distal end of the needle shield 54. For instance, the gripping member 43 may engage an axial gap (not shown) between a proximal end of the needle shield 54 and a distal shoulder of the barrel 52 of the medical container 51, or may engage a recess 552, see for instance Figure 10E, arranged at the distal end of the needle shield 54.

With reference to Figures 9A-9D, the gripping member 43 includes an inward radial tab or hook 431 arranged at a distal end of a radially movable leg 432 for engaging the recess 552 at the distal end of the needle shield 54. The cap 4 may include two diametrically oppposite gripping members 43. The gripping members 43 are radially movable between between a gripping position, in which they grip the needle shield 54, and a release position, outwardly radially located with respect to the gripping position, in which they release the needle shield 54 so that the needle shield 54 can be ejected. Movement of the gripping members 43 is caused by one or more release elements 44 activated by the user. The release elements 44 may be axial levers. The axial levers may be proximally protruding at a inner end of a radial arm 440, while the gripping members 43 may be distally protruding at the inner end of the radial arm 440. The radial arm 440 radially inwardly extends from an outer flange 470 which delimits a proximal stop 47 whose function will be described below. The radial arm 440 forms a resiliently deformable connecting member for connecting the gripping member 43 to the rest of the cap 4. The release elements 44 have a handling feature 441, such as an outer pressing surface. The release elements 44 are radially movable between a first (initial) position, Figure 9B, in which they are not pressed by the user, and an ejection position, Figure 9C, radially inwardly located with respect to the first position, in which they are pressed by the user to allow for ejection of the needle shield 54 outside the cap 4. Movement of the release elements 44 from the first to the ejection position is caused by the user radially inwardly pressing against the outer pressing surfaces 441. Movement to the first position may be caused by the resiliently deformable protrusion returning back towards its rest state. Radial inward movement of the release elements 44 to the ejection position here entails radial outward movement of the gripping members 43 to their release position.

With reference to Figures 10A-10F, the gripping member 43 includes an inward radial tab or hook 431 arranged at an intermediate portion of a radially movable leg 432 for engaging the recess 552 at the distal end of the needle shield 54. The cap 4 may include two diametrically oppposite gripping members 43. The gripping members 43 are radially movable between between a gripping position, Figure 10E, in which they grip the needle shield 54, and a release position, Figure 10F, outwardly radially located with respect to the gripping position, in which they release the needle shield 54 so that the needle shield 54 can be ejected. Movement of the gripping members 43 is caused by one or more release elements 44 activated by the user. The release element 44 may include a cam 444 proximally protruding from a distal end 422 of the cap. The cam 444 is arranged between the distal ends of the two diametrically opposite gripping members 43. The cam 444 may have a non-circular shape, such as an oblong shape.

Stil with reference to Figures 10A-10F, the release element 44 has a handling feature 441, such an outer surface configured for being handled by the user. The release element may have a non-circular shape, such as a square or rectangular shape. The release elements 44 are rotationally movable between a first position, Figure 10E, in which the cam 444 lets the gripping members 43 in their gripping position, and an ejection position, Figure 10F, at 90° from the first position, in which a larger diameter DC of the cam 444 extends between the gripping members 43 such that the gripping members 43 are radially outwardly moved away from each other, thereby reaching their release position. Movement of the release element 44 from the first to the ejection position is caused by the user rotating the release element 44 around the longitudinal axis A with respect to the attachment portion 41 of the cap. Movement of the release element 44 from the ejection to the first position may be either caused by the user rotating the release element 44 back around the longitudinal axis A with respect to the attachment portion 41 of the cap, so that the release element 44 has two stable positions, or by a torsion spring (not shown), so that the release element 44 is monostable, the single stable position being the first position. Rotation of the release element 44 may be guided by a guiding portion 45 of the cap, which may have a cylindrical shape complementary to the shape of an inner cavity of the release element 44. The guiding portion 45 may include one or more circumferential slots 451 for accommodating a hook of a snap-fit member 443 of the release element 44, the snap-fit member 443 axially securing the release element 44 with respect to the guiding portion 45 of the cap.

With reference to Figures 11A-11C, the gripping member 43 includes an inward radial tab or hook arranged at an intermediate portion of a radially movable leg 432 for engaging the recess 552 at the distal end of the needle shield 54. The cap 4 may here include two pairs of diametrically oppposite gripping members 43. The gripping members 43 are radially movable between between a gripping position, Figure 11B, in which they grip the needle shield 54, and a release position, outwardly radially located with respect to the gripping position, in which they release the needle shield 54 so that the needle shield 54 can be ejected. Movement of the gripping members 43 is caused by one or more release elements 44 activated by the user. The release element 44 may include a cam 444 proximally protruding from a distal end 422 of the cap. The cam 444 is arranged between the distal ends 434 of the two diametrically opposite gripping members 43. The cam 444 may have a cylindrical shape.

Still with reference to Figures 11A-11C, the release element 44 has a handling feature 441, such an outer surface configured for being handled by the user, this outer surface including axial handling ribs 442 for easing rotation of the release element 44 with respect to the outer body 2. The release element 44 may have a cylindrical shape. The release element 44 is rotationally and axially movable between a first position, in which the cam 444 lets the gripping members 43 in their gripping position, and an ejection position, at 90° from the first position and proximal to the first position, in which the cam 444 abuts against ramp surfaces 433 provided at the distal end of the gripping members 43 such that the gripping members 43 are radially outwardly moved away from each other, thereby reaching their release position. Movement of the release element 44 from the first to the ejection position is caused by the user rotating the release element 44 around the longitudinal axis A with respect to the attachment portion 41 of the cap 4. Movement of the release element 44 from the ejection to the first position may be either caused by the user rotating the release element 44 back around the longitudinal axis A with respect to the attachment portion 41 of the cap, so that the release element 44 has two stable positions, or by a torsion spring (not shown), so that the release element 44 is monostable, the single stable position being the first position. Rotation of the release element 44 may be guided by a guiding portion 45 of the cap, which may have a cylindrical shape complementary to the shape of an inner cavity of the release element 44. The guiding portion 45 may include one or more helical slots 452 for accommodating a hook of a snap-fit member of the release element 44, such that the movement of the release element 44 with respect to the cap 4 is simultaneously axial and rotational.

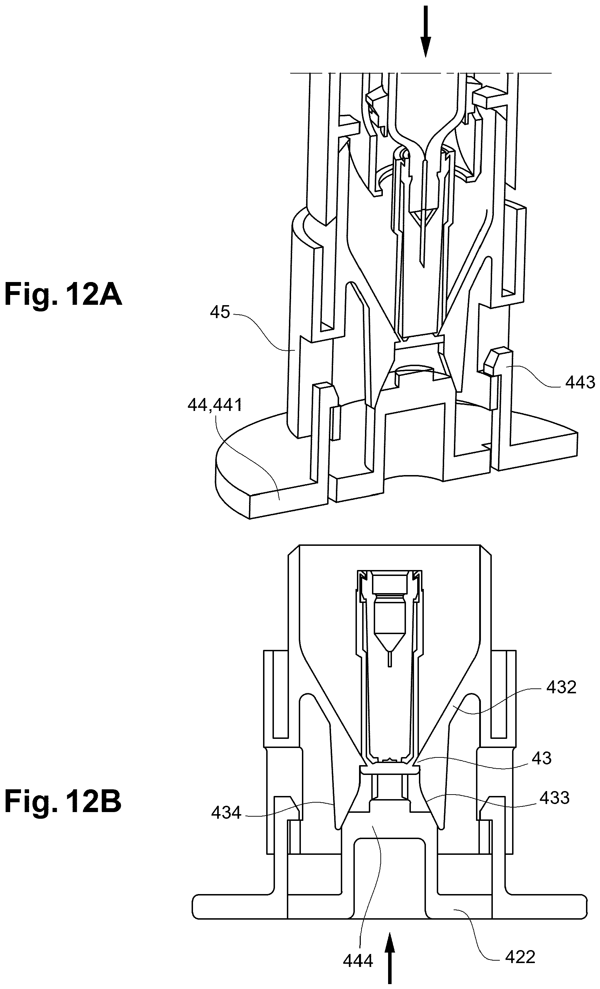

Turning now to Figures 12A-12B, the gripping member 43 includes an inward radial tab or hook 431 arranged at an intermediate portion of a radially movable leg 432 for engaging the recess 552 at the distal end of the needle shield 54. The cap 4 may include two pairs of diametrically oppposite gripping members 43. The gripping members 43 are radially movable between between a gripping position, Figure 12B, in which they grip the needle shield 54, and a release position, outwardly radially located with respect to the gripping position, in which they release the needle shield 54 so that the needle shield 54 can be ejected. Movement of the gripping members 43 is caused by one or more release elements 44 activated by the user. The release element 44 may include a cam 444 proximally protruding from a distal end 422 of the cap. The cam 444 is arranged between the distal ends 434 of the two diametrically opposite gripping members 43. The cam 444 may have a cylindrical shape.

The release element 44 has a handling feature 441, such an outer flange and/or a distal pushing surface to allow handling of the release element 44 by the user. The release element 44 here is axially movable between a first position, in which the cam 444 lets the gripping members 43 in their gripping position, and an ejection position, proximal to the first position, in which the cam 444 abuts against ramp surfaces 433 provided at the distal end 434 of the gripping members 43 such that the gripping members 43 are radially outwardly moved away from each other, thereby reaching their release position. Movement of the release element 44 from the first to the ejection position is caused by the user moving the release element 44 in the proximal direction along the longitudinal axis A. Movement of the release element 44 from the ejection to the first position may be caused by the user. Axial movement of the release element 44 may be guided by a guiding portion 45 of the cap, which may include one or more windows for accommodating a hook of a snap-fit member 443 of the release element 44.

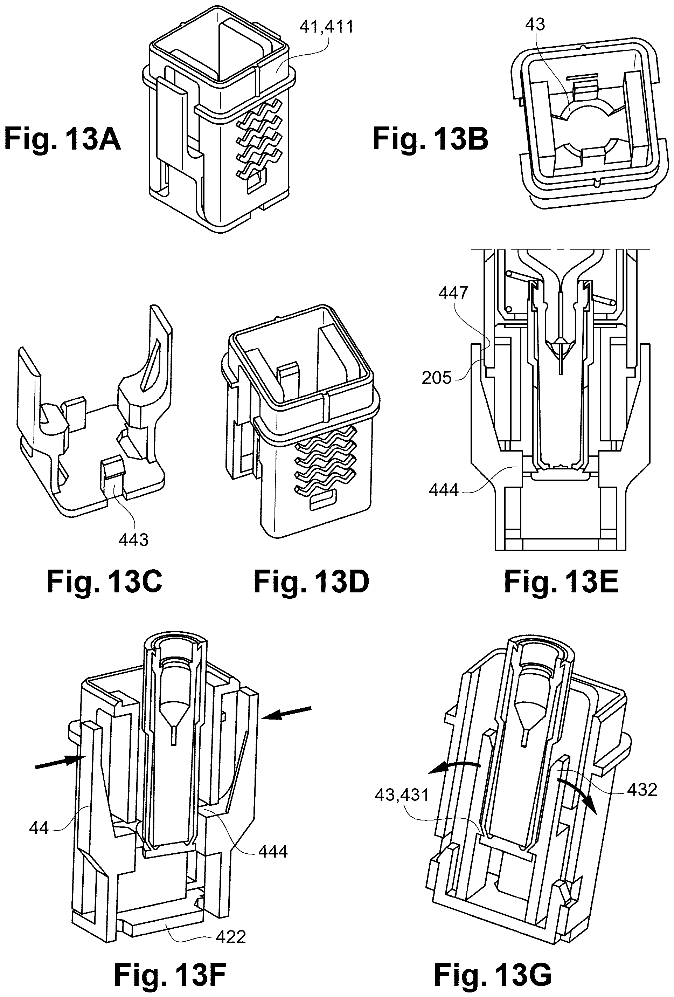

With reference to Figures 13A-13G, the gripping member 43 includes an inward radial tab or hook 431 arranged at an intermediate portion of a radially movable leg 432 for engaging the recess 552 at the distal end of the needle shield 54. The cap 4 may include two diametrically oppposite gripping members 43. The gripping members 43 are radially movable between between a gripping position, in which they grip the needle shield 54, and a release position, outwardly radially located with respect to the gripping position, in which they release the needle shield 54 so that the needle shield 54 can be ejected. Movement of the gripping members 43 is caused by one or more release elements 44 activated by the user. The release element 44 may include one or more radial cams 444 provided at two proximally protruding legs axially protruding from a distal end 422 of the cap 4. The cams 444 are configured to extend between the proximal ends of the two gripping members 43, i.e. proximal to the inward radial tabs.

The release element 44 here is rotationally and axially fixed with respect to the attachment portion 41 of the cap, and may be attached to the rest of the cap 4 by one or more snap-fit members 443. The proximally protruding legs of the release element 44 are radially movable between a first position, Figure 13E, in which their cams 444 let the gripping members 43 in their gripping position, and an ejection position, radially inwardly located with respect to the first position, in which the cam 444 abuts against the gripping members 43 such that the gripping members 43 are radially outwardly moved away from each other, thereby reaching their release position. Movement of the proximally protruding legs from the first to the ejection position is caused by the user radially inwardly pushing against an outer surface of these proximally protruding legs. Movement of the release element 44 from the ejection to the first position may be due to the resiliency of the proximally protruding legs.

In order to prevent release of the needle shield during removal of the cap from the body, the release element 44 may include a radial blocking surface 447, Figure 13E, which may be arranged at an inner side of the proximal end of the proximally protruding legs for radially abutting against a corresponding blocking surface 205 of the outer body 2, this blocking surface 205 of the outer body 2 being defined by the lateral wall 20 of the outer body 2 at a distal end thereof. Thus, as long as the cap 4 is attached to the outer body 2, the blocking surfaces 205, 447 prevent movement of the release element 44 to the ejection position.

With reference to Figures 14A-14I, the gripping member 43 includes an inward radial tab or hook 431 arranged at a distal end of a radially movable leg 432 for engaging the recess 552 at the distal end of the needle shield 54. The radially movable leg 432 here distally extends from a sleeve 414 proximally protruding at the distal end of the attachment portion 41 of the cap. The sleeve 414 is configured for accommodating the needle shield 54. The cap 4 may include two diametrically oppposite gripping members 43. The gripping members 43 are radially movable between between a gripping position, in which they grip the needle shield 54, and a release position, outwardly radially located with respect to the gripping position, in which they release the needle shield 54 so that the needle shield 54 can be ejected. Movement of the gripping members 43 is caused by one or more release elements 44 which here are indirectly activated by the user. By indirectly it is meant that the user here does not directly handle the release element 44. The release element 44 may include a cylindical ring including an inner cavity 445 configured to receive the gripping members 43, an inner wall of this inner cavity having a non-circular cross-section shape, such as a ellipse shape.

The release element 44 has a driving feature 446, such an axial protrusion at a proximal end of this release element 44, for causing rotation of the release element 44. The release element 44 is rotationally movable between an ejection position, in which the gripping members 43 are located in the large diameter portion LD of the inner cavity 445 defined by the inner wall, and a first position, 90° from the ejection position, in which the which the gripping members 43 extend in a small diameter portion SD of the inner cavity 445 such that the inner wall of the inner cavity 445 inwardly radially presses against the gripping members 43 which are forced to their gripping position. Movement of the release element 44 from the ejection to the retaining position is caused by the user rotating the locker 3 from the release to the locking position. The casing 1 indeed includes a motion transmitter 46 arranged within the outer body 2, and between the locker 3 and the release element 44, for transmitting a torque exerted by the user on the locker 3 to the release element 44. Movement of the release element 44 from the retaining to the ejection position may be directly caused by the user.

The motion transmitter 46 may be in the form of an axial sleeve arranged between the outer body 2 and the injection device 5. The motion transmitter 46 has a distal end 461 which may be provided with a notch 462 complementarily engaged by the driving feature 446 of the release element 44, such that rotation of the motion transmitter 46 in one way or the other entails simultaneous rotation of the release element 44 in the same way. The motion transmitter 46 has a proximal end 463 provided with a radial flange 464 configured to axially lean against the flange 21 of the outer body 2. The radial flange 464 may include circular slots 465 engaged by a proximal protrusion 212 protruding from the flange 21 of the outer body 2 to guide rotation of the motion transmitter 46 around the longitudinal axis A. The motion transmitter 46 is indeed rotationally movable with respect to the outer body 2 between a first angular position, corresponding to a release position of the locker 3, and a second angular position, 90° from the first angular position, corresponding to the locked position of the locker 3. In the first angular position of the motion transmitter 46, the release element 44 may be in its ejection position. In the second angular position of the motion transmitter 46, the release element 44 may be in its first position. Rotation of the motion transmitter 46 from the first angular position to the second angular position, or vice versa, is caused by the locker 3 being rotated by the user. Indeed, the locker 3 may have a driving feature 332, such as an axial plate, for complementarily engaging a driving feature 466 of the motion transmitter 46, such as an axial slot, such that rotation of the locker 3 in one way or the other entails simultaneous rotation of the motion transmitter 46 in the same way.

The cap 4 may include a proximal stop 47 configured for axially abutting against the outer body 2 so that attachment of the cap 4 to the outer body 2 is stopped at a predetermined axial position. This enables to make sure that the gripping member 43 of the cap 4 does grip the needle shield 54 of the injection device 5. In the embodiment illustrated in Figure 9A, the proximal stop 47 is a proximal side of the outer flange 470 arranged between the attachment portion 41 and the gripping member. As shown in Figure 10C, the proximal stop 47 may be a peripheral protrusion or rib outwardly protruding from the sidewall of the cap 4. In the embodiment illustrated in Figure 11B, the proximal stop 47 is the proximal end 421 of the cap. In another embodiment illustrated in Figure 14B, the proximal stop 47 may be a proximal shoulder separating the attachment portion 41 from the rest of the cap, and more specifically from a distal housing portion that accommodates the release element 44.

In some embodiments, the cap 4 may include centering features 48 for centering and guiding the needle shield 54 with respect to the cap, so that the needle shield 54 properly engages the gripping member 43 of the cap. As illustrated in Figure 10D, the centering features 48 may include one or more, for instance two diametrically opposite axial ribs protruding from a lateral wall of the cap 4. The axial ribs are arranged at 90° with respect to the two diametrically opposite gripping members 43.

It is contemplated that the cap 4 may be made of a single piece, as illustrated for instance in Figures 9A-9D, in which the release element 44, the gripping member, and the attachment portion 41 form a single part. In other embodiments, the cap 4 may be made of two or more assembled distinct parts. For instance, as illustrated in Figures 10A-10F, 11A-11C, 12A-12B, 13A-13G, and 14A-14I, the release element 44 and the attachment portion 41 of the cap 4 are two distinct parts of the cap. In most embodiments, the gripping member 43 and the attachment portion 41 of the cap 4 may be made of a single piece.

Operation of the casing 1 according to the embodiment of Figures 3A-3B will now be described with reference to Figures 15A-15F.

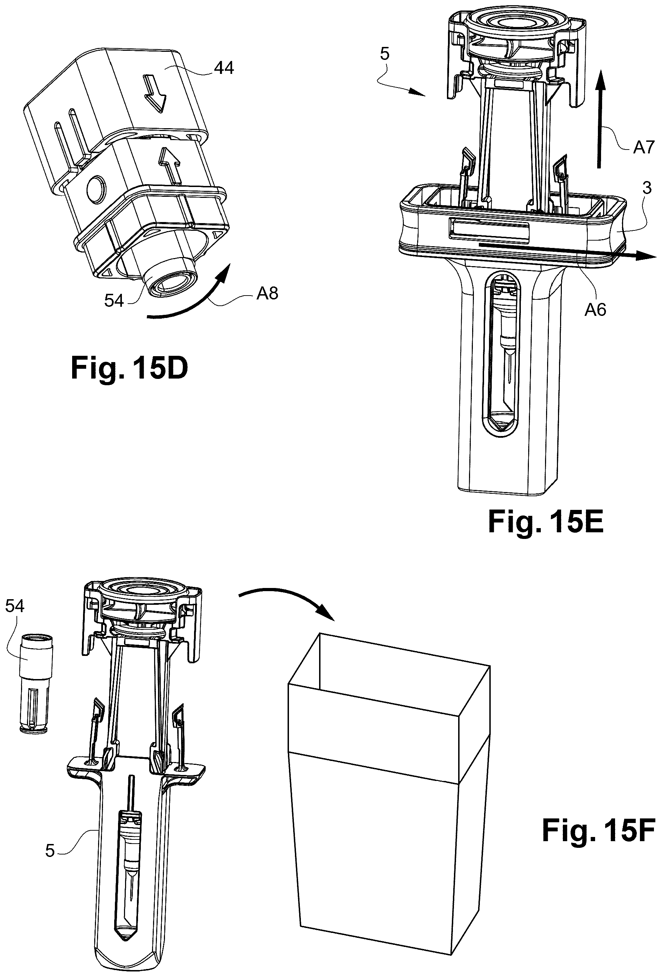

If need be, the user first moves the locker 3 to the release position, see arrow A1 in Figure 15A. The locker 3 may then insert the injection device 5 within the casing 1, as illustrated by arrow A2 in Figure 15B. During insertion, the needle shield 54 of the injection device 5 gets engaged with the gripping member 43 of the cap. Once insertion of the injection device 5 is stopped by the proximal stop 22 of the casing 1, the user can move the locker 3 back to the locked position, see arrow A3 in Figure 15B. As a result, the injection device 5 is secured within the casing 1. The user then removes the cap 4 by distally pulling the cap 5 off the casing 1, as illustrated by the arrow A4 in Figure 15B. Due to the gripping member 43 engaged with the needle shield 54, the needle shield 54 is removed too. The user can thus perform injection. The distal end 202 of the outer body 2 abuts against the injection site, which ensures to prick at a predetermined injection depth. The user pushes the plunger rod 55 in the distal direction, arrow A5 in Figure 15C, so that the medical product contained within the injection device 5 is expelled towards the injection site via the injection needle 53. At the end of injection, the plunger rod 55 moves the trigger fingers 621 apart, thereby triggering deployment of the needle guard 62 which surrounds the injection needle 53 to protect the user against needle stick injuries. The user moves the locker 3 to the release position, see arrow A6 in Figure 15E. The used injection device 5 can thus be withdrawn from the casing 1, arrow A7 in Figure 15E. The user also moves the release element 44 to its ejection position, see arrow A8 in Figure 15D. This causes the gripping member 43 to release the needle shield 54. The used injection device 5 and needle shield 54 can then be discarded in an appropriate waste container, Figure 15F. The user may re-attach the cap 4 to the outer body 2 of the casing 1 so that the casing 1 can be re-used with another injection device 5.

It is readily understandable from the above description that the casing 1 of the invention can thus be re-used instead of being discarded. This improves sustainability. Besides, the casing 1 allows for a better handling by the user and enables to control the needle exposure length and by extension the injection depth without requiring a high accuracy from the user. The casing 1 is therefore easier to use. It is to be understood that the present invention is not limited to the embodiments described above and illustrated in the drawings; rather, the skilled person will recognize that many changes and modifications may be made within the scope of the appended claims.

## Claims

1. Casing (1) configured for receiving an injection device (5), the casing (1) including:
a body (2) delimiting an inner cavity for receiving the injection device (5), the body (2) including a proximal end (204) and a distal end (202) configured for axially abutting against an injection site,
a proximal stop (22) configured for blocking distal movement of the injection device (5) at a predetermined axial position within the body (2),
a locker (3) for axially blocking the injection device (5) within the body (2), the locker (3) being movable with respect to the body (2) between an unlocked position allowing insertion or removal of the injection device (5), and a locked position in which the locker (3) prevents removal of the injection device (5) from the body (2), and
a cap (4) removably attached to the body (2), the cap (4) including a gripping member (43) configured for gripping a needle shield (54) of the injection device (5), such that removal of the cap (4) from the body (2) entails removal of the needle shield (54) from the injection device (5), and a release element (44) configured for releasing the needle shield (54).

2. Casing (1) according to the preceding claim, wherein the proximal stop (22) is arranged at the proximal end (204) of the body (2).

3. Casing (1) according to any of the preceding claims, wherein the body (2) includes a finger flange (21) for providing support to a user's fingers.

4. Casing (1) according to the preceding claim, wherein the finger flanger (21) includes a guiding member (24) for guiding movement the locker (3).

5. Casing (1) according to any of the preceding claims, wherein the locker (3) is radially, rotationally or axially movable with respect to the body (2).

6. Casing (1) according to any of the preceding claims, wherein the cap (4) is made of a single piece.

7. Casing (1) according to any of the preceding claims 1-5, wherein the cap (4) is made of two assembled parts: (i) an attachment portion (41) configured to be removably attached to the body (2), and (ii) the release element (44).

8. Casing (1) according to the preceding claim, wherein the gripping member (43) is movable between a gripping position in which the the gripping member (43) can grip the needle shield (54) of the injection device (5) and a release position in which the gripping member (43) can release the needle shield (54).

9. Casing (1) according to the preceding claim 7 or 8, wherein the release element (44) is movable between a first position in which the release member allows the gripping member (43) to grip the needle shield (54) and an ejection position in which the release member moves the gripping member (43) to the release position.

10. Casing (1) according to any of the preceding claims 9-11, wherein the release element (44) is rotationally movable with respect to the gripping member (43).

11. Casing (1) according to any of the preceding claims 9-11, wherein the release element (44) is axially movable with respect to the gripping member (43).

12. Casing (1) according to any of the preceding claims 9-11, wherein the release element (44) is both rotationally and axially movable with respect to the gripping member (43).

13. Casing (1) according to any of the preceding claims 9-11, wherein the release element (44) is radially movable with respect to the gripping member (43).

14. Casing (1) according to any of the preceding claims when dependent on claim 8, wherein the casing (1) includes a motion transmitter (46) arranged between the locker (3) and the gripping member (43) such that movement of the locker (3) from the unlocked position to the locked position entails movement of the gripping member (43) from the release position to the gripping position.

15. Drug delivery device including a casing (1) according to any of claims 1-14 and an injection device (5) arranged within the casing (1).
